(19) Europäisches Patentamt
European Patent Office
Office européen des brevets

(11) **EP 4 585 194 A1**

(12) **EUROPEAN PATENT APPLICATION**

(43) Date of publication:
**16.07.2025 Bulletin 2025/29**

(21) Application number: **24382019.8**

(22) Date of filing: **11.01.2024**

(51) International Patent Classification (IPC):
**A61F 11/00** (2022.01)    **A61L 27/20** (2006.01)
**A61L 27/22** (2006.01)

(52) Cooperative Patent Classification (CPC):
(C-Sets available)
**A61L 27/222; A61L 27/20;** A61F 11/20;
A61L 2430/14                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Administración General de la Comunidad
Autónoma de Euskadi (AGCAE)
01010 Vitoria-Gasteiz (Álava) (ES)**
• **Universidad Del Pais Vasco-Euskal
Herriko Unibertsitatea
48940 Leioa (Bizkaia) (ES)**

(72) Inventors:
• **IZETA PERMISÁN, Ander
20014 SAN SEBASTIÁN (Gipuzkoa) (ES)**
• **CHIESA ESTOMBA, Carlos
20014 SAN SEBASTIÁN (Gipuzkoa) (ES)**
• **IRASTORZA LORENZO, Ainhoa
20014 SAN SEBASTIÁN (Gipuzkoa) (ES)**
• **DE LA CABA CIRIZA, Koro
20018 DONOSTIA (Gipuzkoa) (ES)**
• **GUERRERO MANSO, Pedro
20018 DONOSTIA (Gipuzkoa) (ES)**

(74) Representative: **Herrero & Asociados, S.L.
Edificio Aqua - Agustín de Foxá, 4-10
28036 Madrid (ES)**

(54) **BIOMATERIAL FILM, USES AND PREPARATION METHOD THEREOF**

(57)    The present invention refers to a biomaterial film comprising a protein component, in a concentration comprised between 2 and 8 % by weight, monosaccharides/-disaccharides, in a concentration comprised between 0.5 and 2 % by weight, agar, in a concentration comprised between 0.1 and 1 % by weight, and glycerol, in a concentration comprised between 0.2 and 1.5 % by weight, wherein the film has a thickness comprised between 70-150 µm and a rugosity lower than 70 GU. The invention also refers to a method for preparing said biomaterial film. The use of the biomaterial film in medicine, in particular, in the treatment of chronic tympanic membrane perforation is also contemplated. Finally, the present invention refers to an implant comprising the biomaterial film of the invention.

EP 4 585 194 A1

(52) Cooperative Patent Classification (CPC): (Cont.)

C-Sets
**A61L 27/20, C08L 5/12**

**Description**

Field of the invention

The present invention belongs to the field of tissue engineering, in particular to a biomaterial film suitable for wound healing applications. More particularly, the present invention refers to a tympanic membrane implant comprising the biomaterial film for use in the treatment of chronic perforations of the tympanic membrane.

Background of the invention

The tympanic membrane (TM) or commonly, eardrum, is a thin tissue that separates the external auditory canal from the middle ear. Its principal function is the transference of soundwaves into mechanical vibrations from the ear canal into the middle ear (Groen JJ. Functional Anatomy of Hearing. Acta Otolaryngol (Stockh). 1951;40(1-2):32-41). The TM consists of three main parts: i) pars tensa, which covers the major portion of the TM and is primarily involved in the transmission of sound vibrations, ii) pars flaccida, involved in balancing ear pressure differences, and iii) the handle of the malleus, which connects internally with the bony ossicles (Lim DJ. Structure and function of the tympanic membrane: a review. Acta Otorhinolaryngol Belg. 1995;49(2):101-115). The anatomical structure of the TM is composed of three layers: i) an epithelium, in external side, ii) two layers of connective tissue, composed mainly of collagen type I, II and III, arranged radially and circularly, and iii) a mucosal membrane that represents the internal side into the middle ear (Knutsson J, et al. Collagen Type Distribution in the Healthy Human Tympanic Membrane. Otol Neurotol. 2009;30(8):1225-1229; Lim DJ. Human Tympanic Membrane: An Ultrastructural Observation. Acta Otolaryngol (Stockh). 1970;70(3):176-186; Schmidt SH, et al. Tympanic-Membrane Structure - New Views. ORL. 1991;53(1):32-36.; Stenfeldt K, et al. The Collagen Structure of the Tympanic Membrane: Collagen Types I, II, and III in the Healthy Tympanic Membrane, During Healing of a Perforation, and During Infection. Arch Otolaryngol Neck Surg. 2006;132(3):293.).

Perforations of the tympanic membrane (PTMs) are one of the most common hearing illnesses, being trauma and infections the principal causes. The regeneration depends mainly on the size and location of the perforations (Dolhi N. et al. Tympanic Membrane Perforations. In: StatPearls. StatPearls Publishing; 2023. Accessed June 1,2023.http://www.ncbi.nlm.nih.gov/books/NBK557887/). Most of them close spontaneously by a process led by the epithelium which, from the margins, is able to close the perforation, after which the new fibrous and mucosal layers are formed (Gladstone HB, et al. Tympanic membrane wound healing. An overview. Otolaryngol Clin North Am. 1995; 28(5):913-932; Santa Maria PL, et al. Histology of the healing tympanic membrane following perforation in rats: Tympanic Membrane Healing Histology. The Laryngoscope. 2010;120(10):2061-2070). This inherent regenerative capacity has been attributed to the presence of latent progenitor cells within the epithelial layer at the malleus and annulus regions of the TM (Kim SW, et al. Latent progenitor cells as potential regulators for tympanic membrane regeneration. Sci Rep. 2015;5(1):11542). However, some of the perforations may become chronic (cPTMs) due to large size wounds, recurrent infections, or poor healing capacity, showing up as ear infections, otalgia, cholesteatoma formation and/or hearing loss (Jellinge ME, et al. Spontaneous closure of traumatic tympanic membrane perforations: observational study. J Laryngol Otol. 2015;129(10):950-954; Masaki M, et al. Experimental Cholesteatoma: Epidermal Ingrowth through Tympanic Membrane following Middle Ear Application of Propylene Glycol. Acta Otolaryngol (Stockh). 1989;108(1-2):113-121; Orji FT et al. Determinants of spontaneous healing in traumatic perforations of the tympanic membrane. Clin Otolaryngol. 2008;33(5):420-426). These cases require reconstructive surgical treatments that allow the formation of the native structure, as the cPTMs lack the support to facilitate the necessary cell migration and proliferation.

The current treatment for induction of regeneration, called myringoplasty, is based on the use of autologous tissues such as temporalis muscle fascia or tragal cartilage perichondrium to support cell migration and wound closure (Dvorak DW, et al. Repair of chronic tympanic membrane perforations with long-term epidermal growth factor: Tympanic Membrane Repair. The Laryngoscope.1995;105(12):1300-1304). Although it is a successful technique in most cases, the operation per se involves risk, specialised surgical skills, high cost and post-operative discomfort. Moreover, recurrent perforations remain frequent due to ear pressure differences and infections (Aleemardani M, et al. Can Tissue Engineering Bring H-ope to the Development of Human Tympanic Membrane? Tissue Eng Part 8 Rev. 2021;27(6):572-589; Hardman J, et al. Tympanoplasty for Chronic Tympanic Membrane Perforation in Children: Systematic Review and Meta-analysis. Otol Neurotol. 2015;36(5):796-804), so that, given the limited availability of autologous graft tissue, the clinician is often left with insufficient material for successive tympanoplasties (Aleemardani M, et al. 2021).

Tissue engineering has emerged as a promising alternative for this type of pathology that requires reconstructive treatments. Many attempts have been made to design biomaterials that serve as structural support to guide the closure of cPTMs, in which cellular or acellular constructs have been used, based on both natural (decellularised tissues, chitosan, alginate, hyaluronic acid) or synthetic materials (PLA, PLGA, PCL), and processed using different manufacturing techniques (Ghanad I, et al. A Systematic Review of Nonautologous Graft Materials Used in Human Tympanoplasty. The Laryngoscope. 2021; 131(2):392-400; Guruswamy Damodaran R, et al. Tissue and organ decellularization in regenerative medicine. Biotechnol Prog. 2018;34(6):1494-1505; Sainsbury E, et al. Tissue engineering and regenerative medicine strategies for the repair of tympanic membrane perforations. Biomater Biosyst. 2022;6:100046).

Despite the presence of tissue engineering-based commercial products, like ClearDrum, TymCure, or Phonograft, scaffolds that can replace the gold standard technique have not been developed yet, since they lack biomimetic structure or appropriate biomechanical properties, and professionals still resort to myringoplasty due to the limitations associated with the existing tissues engineering-based options.

Furthermore, most of commercial scaffolds still involve implantation techniques that require hospitalisation of the patient and great surgical skills (Hussain Z. et al. Necessities, opportunities, and challenges for tympanic membrane perforation scaffolding-based bioengineering. Biomed Mater. 2021;16(3):032004).

Thus, there is an unmet clinical need for novel therapies, based on regenerative medicine, that allow the growth of regenerative epithelium, avoiding hospital admission and thus reducing the morbidity associated with current therapies.

Summary of the invention

In a first aspect, the present invention refers to a biomaterial film comprising: A protein component, in a concentration comprised between 2 and 8 % by weight, monosaccharides/disaccharides, in a concentration comprised between 0.5 and 2 % by weight, agar, in a concentration comprised between 0.1 and 1 % by weight, and glycerol, in a concentration comprised between 0.2 and 1.5 % by weight, wherein the film has a thickness comprised between 70-150 $\mu$m and a rugosity lower than 70 GU.

In a second aspect, the present invention refers to a method for preparing the biomaterial film of the invention.

In a third aspect, the invention refers to the use of the biomaterial film in medicine, in particular, in the treatment of chronic tympanic membrane perforation.

Finally, in a fourth aspect, the present invention refers to a tympanic membrane implant comprising the biomaterial film of the invention.

Brief description of the drawings

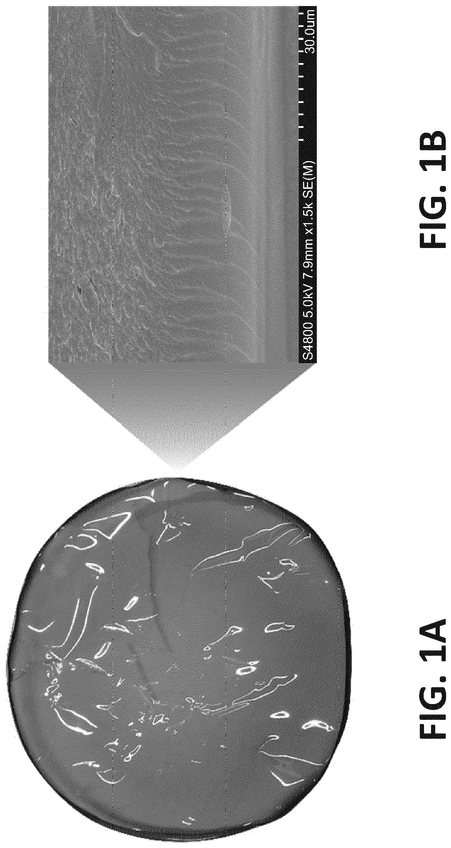

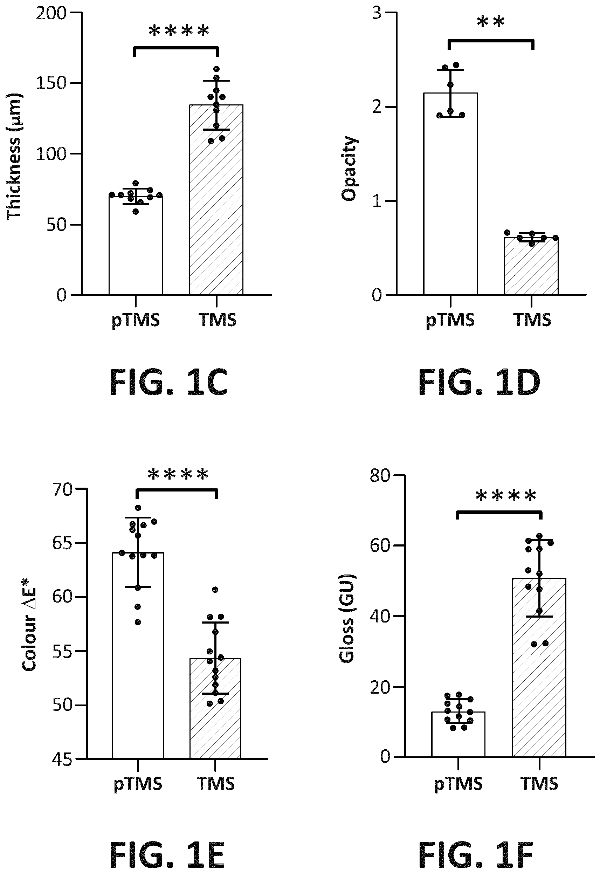

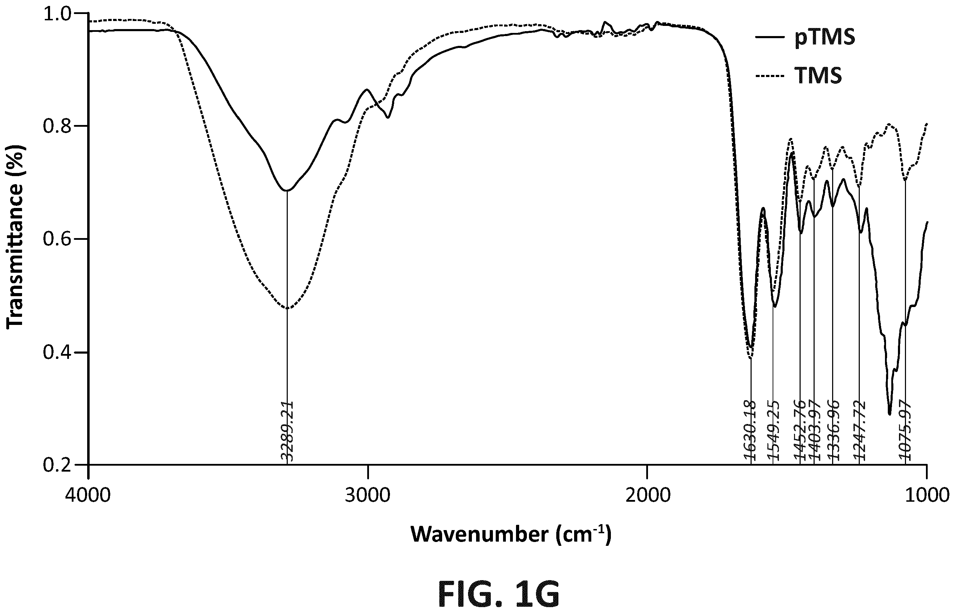

**Figure 1. Physicochemical properties of the film (TMS).** A) Macroscopic appearance of the TMS, B) Microscopic appearance of the TMS through SEM, C) thickness, D) opacity, E) colour, F) gloss, and G) FTIR measurements of the TMS before and after purification and sterilisation.

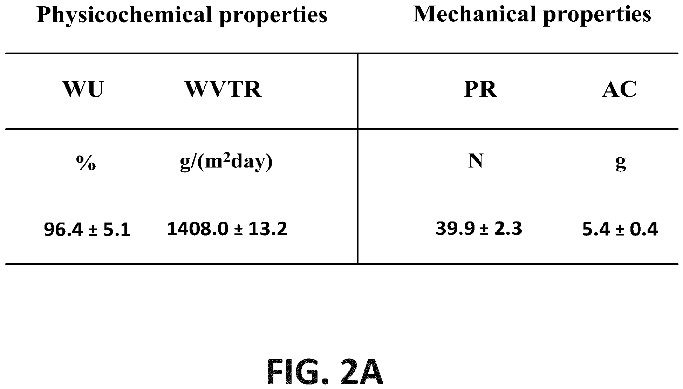

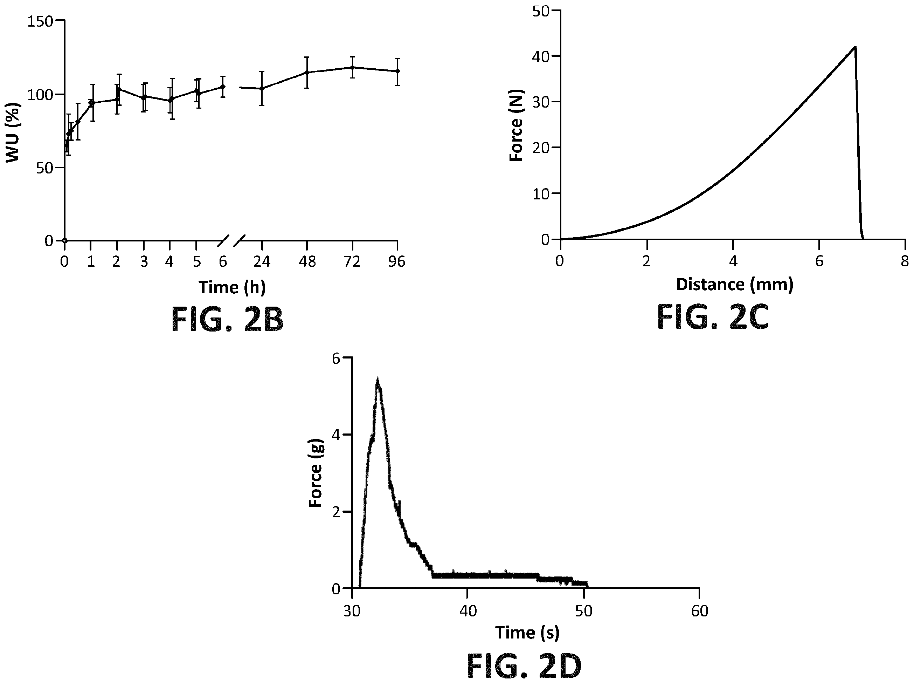

**Figure 2. Physicochemical and mechanical properties of the film (TMS).** Mean values of film performance for A) Water Uptake (WU), Water Vapour Transmission Rate (WVTR), Pressure Resistance (PR), and Adhesion Capacity (AC); B) WU capacity of films over time; Behaviour of one sample in the C) PR and D) AC tests.

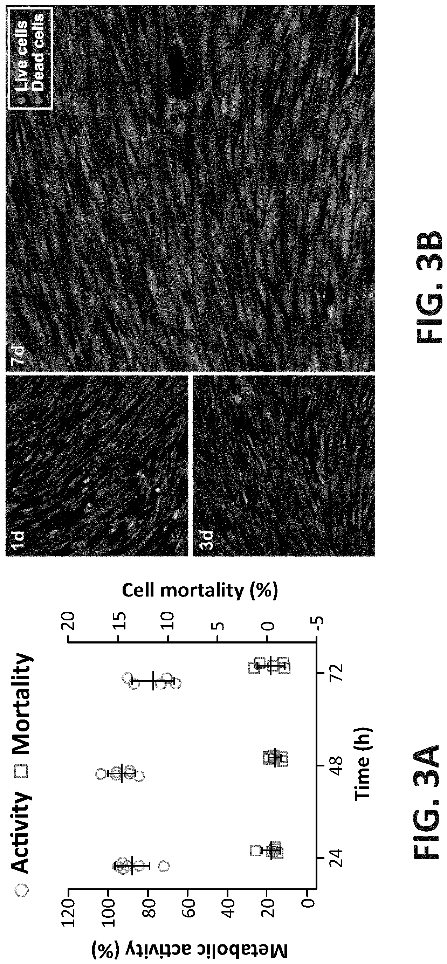

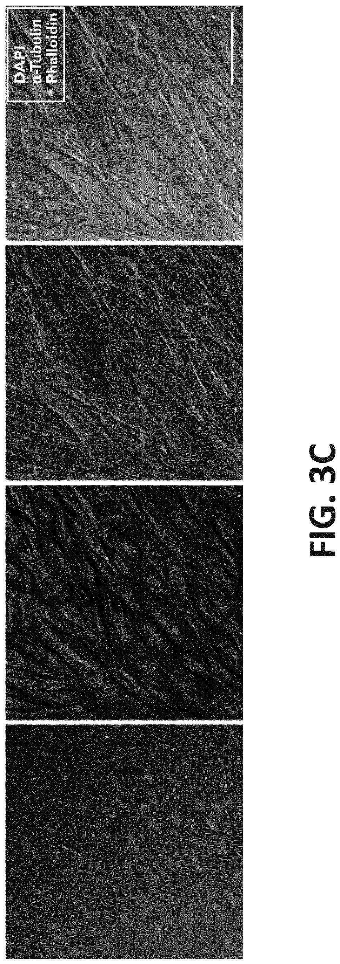

**Figure 3. Cell behaviour analysis with film (TMS) interaction.** Biocompatibility assessment of fibroblasts exposed to the film A) short-term for up to 3 days through cell activity and mortality evaluation and B) long-term for one week with Live/Dead kit. C) Evaluation of cell adhesion after 14 days of culture on the developed material. Immunofluorescence was used to assess the labelling of actin filaments (green) and microtubules (red), nuclei were labelled with DAPI (blue). Scale is 100 $\mu$m.

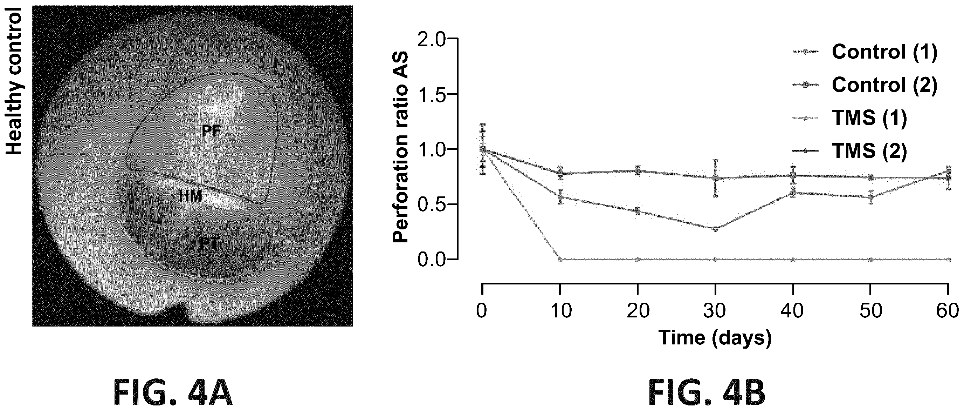

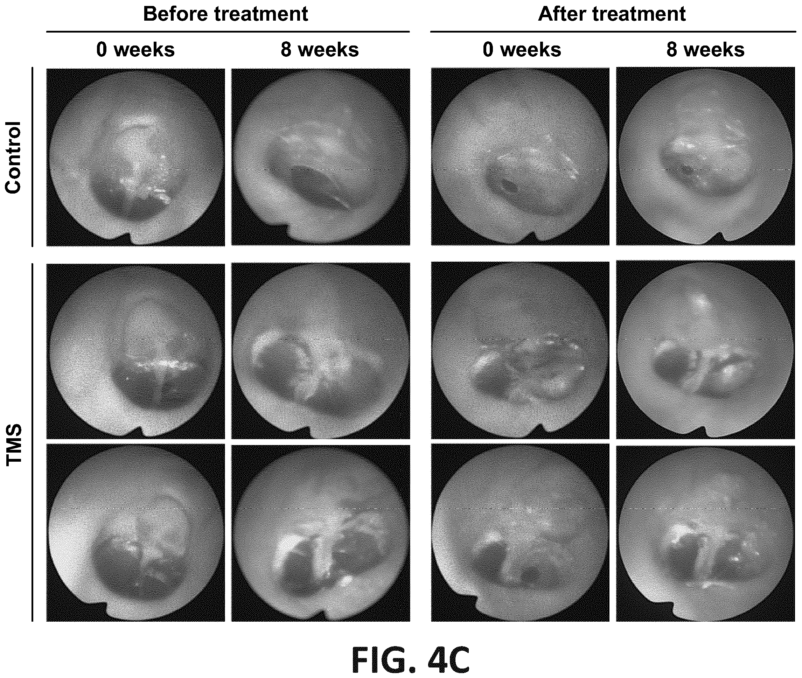

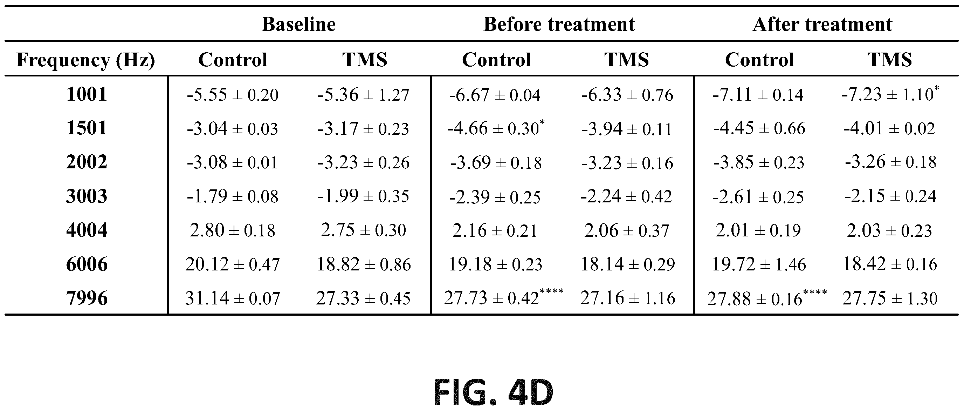

**Figure 4. Otoendoscopic and otoemission evaluation of film (TMS) in vivo implantation.** A) Appearance of a healthy TM showing the main parts (PF: pars flaccida, PT: pars tensa, and HM: handle of the malleus). B) Calculation of the rate of perforation closure after treatment of the control and study groups over time, relativized to the initial day. C) Follow-up of perforations in the chronification (before treatment) and treatment phases of the control and study groups. D) Otoacoustic emissions test mean results for each group at the different frequencies.

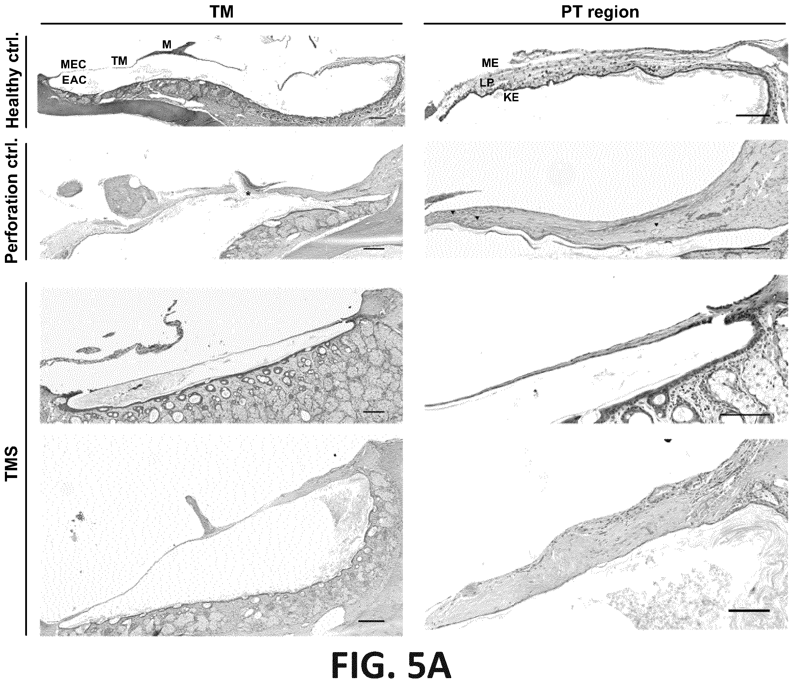

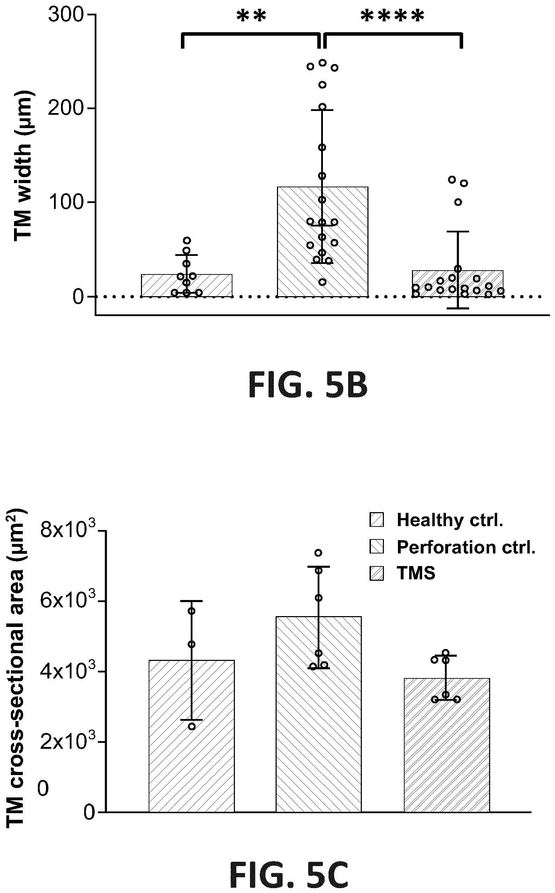

**Figure 5. Histological evaluation of film (TMS) in vivo implantation.** A) Histological samples of healthy tissue, perforation control and graft-treated study group. The general image shows the main structures: external auditory canal (EAC), middle ear cavity (COM), tympanic membrane (TM) and malleus (M). In the images corresponding to the PT region, the mucosal epithelium (ME), lamina propria (LP) and keratinised epithelium (KE) can be distinguished. Perforation is indicated with an asterisk and points of neovascularization with a black arrow. The scale is 100 $\mu$m. Quantification of the B) thickness of the epithelium as well as the C) tympanic cross-sectional area from the histological specimens.

Detailed description of the invention

In response to the clinical needs of novel therapies for the treatment of cPMTs, the present invention presents a new therapeutic solution based on an acellular film composed mainly by protein components and other natural biomaterials.

The authors of the invention have designed a tympanic membrane substitute (TMS) formulated based on proteins, mono- or disaccharides, agar, and glycerol, in adequate concentrations, to achieve a biomaterial film with adequate properties. The formulation has been designed taking into consideration the physicochemical properties that can favour a rapid regenerative process and the mechanical characteristics that can facilitate the implantation of the construct.

The morphological, mechanical, physicochemical and cellular behaviour characterization shows that the film has a thickness in the range of the native TM; it is transparent, which allows the observation of internal structures of the ear, biocompatible and with great stability; it has a great hydration capacity that, added to its good permeability, provides an optimal environment for the regenerative process; it has resistance to pressure, thus allowing it to withstand the pressure differences that occur in the ear and also has great adhesion capacity due to the roughness of its surface, which allow non-invasive implantation.

The healing process was favoured by the specific formulation and properties of the film and, therefore, the film constitutes a TMS that promotes effective and complete closure of the perforation by allowing the growth of the membrane without causing side effects.

The choice of appropriate materials, meeting the outlined criteria and customized for the specific pathology, has facilitated this accomplishment. The materials in this formulation have been precisely selected to serve distinct purposes and provide the material with the most appropriate characteristics to support the regenerative process.

In a first aspect, the present invention refers to a biomaterial film comprising:

- A protein component, in a concentration comprised between 2 and 8 % by weight,
- Mono/disaccharides, in a concentration comprised between 0.5 and 2 % by weight,
- Agar, in a concentration comprised between 0.1 and 1 % by weight, and
- Glycerol, in a concentration comprised between 0.2 and 1.5 % by weight,

wherein the film has a thickness comprised between 70-150 $\mu$m and a rugosity lower than 70 GU (hereinafter, biomaterial film of the invention).

As used herein, the term "biomaterial film" refers to an acellular film made of material or substance composed of components of natural origin that are biocompatible with a human or animal body. The biomaterial film may have been engineered to take a form which, alone or as part of a complex system, may be used to direct, by control of interactions with components of living systems, the course of any therapeutic or diagnostic procedure.

The term "protein component", as used herein, refers to one or more proteins of both vegetal and animal origin.

The expressions "mono/disaccharides" or "monosaccharides or disaccharides", as used herein, refer to one or more monosaccharides, or one or more disaccharides, or different combinations thereof.

In a preferred embodiment, the protein component of the biomaterial film is present in a concentration comprised between 4 and 6% by weight; the mono/disaccharides are present in a concentration comprised between 0.5-1.5% by weight; the agar in a concentration comprised between 0.1-0.5% by weight, and glycerol in a concentration comprised between 0.2-1% by weight.

In a particular embodiment, the biomaterial film of the invention comprises 5% by weight of the protein component; 1% by weight of the mono/disaccharides; 0.25% by weight of agar and 0.5% by weight of glycerol.

In a preferred embodiment, the protein component is gelatin of animal origin. Gelatin is a biomaterial that supports cell-matrix interactions and, thus, cell activity because of its resemblance with native tissues. Preferably, the gelatin is selected from porcine, bovine or marine gelatin, more preferably, porcine gelatin.

The mechanical properties of the film are improved by adding agar, which confers structural support. Additionally, the incorporation of glycerol, as a plasticizer, gave flexibility to the film, simplifying its handling during the implantation procedure.

Mono/disaccharides are added to stabilize the whole formulation through natural crosslinking.

Examples of monosaccharides suitable for the purposed of the present invention can be selected from ribose, fructose, glucose, among others. Examples of disaccharides suitable for the purposed of the present invention can be selected from lactose, sucrose, among others.

In a preferred embodiment, the disaccharide is lactose.

The biomaterial film of the invention can additionally comprise other plasticisers such as sorbitol, polyethylene glycol, or other polysaccharides such as gum arabic, xanthan gum or guar gum.

The film thickness ranges from 70 to 150 $\mu$m, depending on the degree of hydration, closely resembling the thickness of the human eardrum, which varies from around 50 to 120 $\mu$m, depending on the specific anatomical region.

The roughness of the film was determined by measuring the gloss, as these parameters are inversely proportional. Surface roughness fosters greater interaction between cells and the substrate, facilitating mechanical anchoring. This, in turn, triggers the formation of stable focal adhesion points, leading to increased cell growth and proliferation. Cells in direct contact with the material not only adhered to the substrate but also maintained homeostasis, underwent proliferation, and exhibited migration along the construct. The film adhesive properties are adequate to avoid the need of graft suturing, thereby enhancing accessibility for clinicians and minimizing discomfort for patients.

Concerning the mechanical properties of the film, it shows suitable qualities to withstand the differential pressures within the ear canal, as evidenced by the puncture test (see the examples outlined in this application). As previously mentioned, achieving these mechanical properties was made possible through the selection of specific materials composing the formulation. Each of these materials has played a role in supporting the mechanical stability of the film.

The biomaterial film of the invention has structural stability, adopting a configuration that facilitated the movement of water molecules, thus creating an ideal microenvironment in terms of hydration and permeability for wound healing. Furthermore, its surface roughness not only encouraged cell adhesion and proliferation but also facilitated adhesion to the surrounding tissue during implantation, thus reducing the necessity for extensive surgical procedures, sutures, or surgical adhesives.

Another aspect of the invention relates to a method for obtaining the biomaterial film of the invention comprising the steps of:

a) Obtaining an aqueous solution by mixing the protein component, preferably gelatin, in a concentration between 2 and 8 %, preferably between 4 and 6%, and the mono/disaccharides, preferably the disaccharide lactose, in a concentration of 0.5-2%, preferably between 0.5-1.5%, with distilled water, Mili Q or PBS,

b) Immersing the mixture obtained in a) into an oil bath, at 50-90 °C for 15-40 min at 50-500 rpm,

c) Obtaining an aqueous solution by mixing the agar, in a concentration between 0.1 and 1 %, preferably between 0.1 and 0.5%, with distilled water, Mili Q or PBS, and homogenizing for 5-20 min at 95-120 °C,

d) Mixing the solutions obtained in b) and c) and adding glycerol in a concentration between 0.2 and 1.5%, preferably between 0.2 and 1%,

e) Adjusting the pH of the mixture obtained in d) to a pH basic between 8-11, and continuing the homogenization for other 15-40 min at 50-90 °C,

f) Pouring the preparation obtained in e) into moulds, and drying at room temperature for 48-96 h to obtain the films, and

g) Incubating the films obtained in f) for 6-36 h, at 50-120 °C, to induce crosslinking.

In step e) the pH adjustment can be carried out by routine procedures. In particular, this step is based on the addition of NaOH (0.1-10M) in drops while the solution is constantly stirred (with a magnetic stirrer) and the pH is monitored in real time.

In step f) the preparation is preferably poured into Petri dishes (60, 100 or 150 mm in diameter).

Preferably, and prior to its medical use, the films obtained in g) can be purified by dialysis with distilled water for 24-72 h (step h) and sterilized by immersion into 70% ethanol for 5-20 min and exposure to UV radiation (265 nm) for 5-30 min (step i). The purification and sterilisation processes do not induce compound degradation, as evidenced by the absence of by-products release or the formation of new functional groups.

As previously explained, the inventors have shown that the biomaterial films of the invention have physicochemical and biological properties of interest, especially for their application in tissue engineering, in addition to being able to promote wound healing, being applicable in medicine, more particularly in tissue regenerative medicine and/or in the regeneration, repair, or replacement of a subject's tissue.

In the present invention, "subject" is understood to be any animal, preferably a mammal, more preferably a primate, in particular, a human being, of any race, sex or age.

Thus, another aspect of the invention relates to the biomaterial film of the invention, or biomaterial films obtained by the method of the invention, for use in medicine, preferably in regenerative medicine.

Another aspect of the invention relates to the biomaterial film of the invention, or biomaterial films obtained by the method of the invention, for use in the treatment of cPTMs.

As demonstrated in the experimental examples outlined in this application, in studies conducted in animal models of cPTM, the biomaterial film of the invention enables the closure of the tympanic membrane perforation within 10 days of implantation, thereby improving closure rates and time achieved in the state of the art. Animals treated with this tissue engineering substitute exhibit complete regeneration of the tympanic membrane, restoring the native structure, along with complete degradation of the material, without any adverse effects.

This film emerges as an alternative to myringoplasty because it utilizes biocompatible and biomimetic materials already individually employed in other medical products. Consequently, its manufacturing process is simple, safe and cost-effective, as it does not require a significant budget, thereby allowing for easy scalability of production.

The biomaterial film of the invention can be part of an implant. In fact, the biomaterial film can be used for the manufacture of an implant.

Thus, another aspect of the present invention relates to the use of the biomaterial film of the invention for the manufacture of a tympanic membrane implant.

Another aspect of the invention relates to a tympanic membrane implant, hereinafter "the implant of the invention", which comprises the biomaterial film of the invention, as described above. The implant of the invention can additionally comprise other components as chitosan, aloe vera or tetrahydrocurcumin (THC) in order to enhance the mechanical properties of the film, and/or growth factors (EGF, FGF, TGF, etc) and/or cells with regenerative potential (fibroblasts, autologous stem cells, etc.).

The term "implant" refers to any device or tissue that is biocompatible with the physiological state of the subject's body and does not produce adverse side effects, and that is structured, designed or configured to be placed partially or totally on a subject's body temporarily or permanently for one or more therapeutic or prophylactic purposes, such as increasing the tissues, the contour, replacing or supporting the physiological function, repairing or restoring tissue damaged by disease or trauma, and/or administering therapeutic agents to normal, damaged or sick organs and tissues.

The tympanic membrane implant, comprising the film of the invention, behaves as a TMS, constituting an effective support or scaffold for reepithelization and closure of cPMTs, surpassing current treatments (both autologous and non-autologous) in terms of closure rates and time. Furthermore, it enables complete recovery of the native structure and the full degradation of the implanted material without causing any adverse effects.

In a particular embodiment, the tympanic membrane implant of the invention comprises a biomaterial film comprising:

- 5% by weight of gelatin,
- 1 % by weight of lactose
- 0.25 % by weight of agar, and
- 0.5 % by weight of glycerol.

wherein the film has a thickness comprised between 70-150 $\mu$m and a rugosity lower than 70 GU.

Thus, the biofilm or the implant of the invention emerges as an alternative to myringoplasty with the following advantages:

- Utilization of biocompatible and biomimetic materials,
- Simple and cost-effective manufacturing, with easy scalability,
- High stability,
- Non-invasive implantation and absence of need for local anaesthesia,
- Possibility of performing in a clinical consultation,
- Higher closure rates compared to conventional techniques,
- Shorter closure times compared to conventional techniques,
- Recovery of the native structure without adverse effects, complete degradation of the implant.

Therefore, this technology offers not only optimised manufacturing, tailored to the requirements of the condition and individual patients, but also simplifies surgical procedures and reduces productions costs. Therefore, the tympanic membrane implant not only demonstrates superior closure rates compared to conventional techniques but also holds potential as a commercial product. Its ease of production and stability would allow for the off-the-shelf availability in the clinic, simplifying and reducing the time of surgical procedures, while inducing closure of the perforations.

The following examples are not intended to limit the present invention but are by way of illustration.

EXAMPLES

Materials and methods

1. Biomaterial film (TMS) production

Porcine gelatin (type A, 270 bloom) was purchased from Sancho de Borja (Zaragoza, Spain) and agar (4.63 g $SO_4^{2-}$ per kg) was provided by Hai Long Robika Factory (Dong Thap, Vietnam). Glycerol (purity of 99.5%), used as plasticizer, and lactose, used as cross-linker, were obtained from Panreac (Barcelona, Spain). MiliQ water (Merck) was used to prepare the solutions. All chemicals were of pharmaceutical grade, and they were used as received without further purification.

For the preparation of the film, first 5 g (5%) of gelatin and 1 g of lactose were added to 60 mL of MiliQ water. The mixture was immersed into an oil bath at 80°C for 30 min at 200 rpm. Next, 0.25 g (0.25%) of agar were added over 40 mL of MilliQ water and allowed to homogenize for 10 min at 100°C. Subsequently, both solutions were mixed, 0.5 g (0.5%) of glycerol were added, the pH was adjusted to 10, and homogenization was maintained for other 30 min at 80°C. Finally, 8.5 g of the preparation were poured into 60 mm ⌀Petri dishes, which were left to dry at room temperature for 72 h to obtain the films. To complete the film preparation process, the samples were incubated for 24 h at 100°C to induce lactose crosslinking. Prior to the use of the constructs, the samples were purified by dialysis with distilled water for 48 h and sterilized by immersion into 70% ethanol for 5 min and exposure to UV radiation (265 nm) for 15 min. The product obtained before post-processing steps will be referred to as pTMS (pre-TMS), while the final product will be cited simply as TMS. A hand-held QuantuMike digimatic (Mituyoto) micrometer, with an accuracy of 0.001 mm, was used to measure TMS thickness (n= 10) of both samples, before and after sterilisation.

2.TMS physicochemical characterisation

*Scanning electron microscopy*

The inner morphology of the films was analysed by using a field emission scanning electron microscope Hitachi S-4800 (Hitachi High-Technologies Corporation) with an acceleration voltage of 15 kV. For that, samples were mounted on a metal stub and coated under vacuum with gold in an argon atmosphere prior to the observation.

*Colour*

The Konica Minolta Chromometer CR-400 was employed to determine the colour of the film before and after sterilisation (n= 12). The CIELAB scale was used for that aim: L* = 0 (black) to L* = 100 (white), -a* (greenness) to +a* (redness), and -b* (blueness) to +b* (yellowness) using a white control (L* = 97.39, a* = 0.03, and b* = 1.77). The total colour difference (ΔE*), referred to the white control, was determined with the following equation:

$$\Delta E^* = \sqrt{(\Delta L^*)^2 + (\Delta a^*)^2 + (\Delta L^*)^2}$$

*Gloss*

A Konika Minolta Multi gloss 268 plus glossmeter was used to measure gloss in both film conditions (n= 12), with a 60° incidence angle, according to ASTM D-523 method specifications.

*Transparency*

A Multiskan SkyHigh (Thermo Fischer) ultraviolet-visible (UV-Vis) spectrophotometer was used for the measurement of the light absorbance at 600 nm in order to determine the TMS transparency. For that, the opacity value was measured, defined as the value obtained from absorbance at 600 nm divided by the previously determined thickness for each sample. Therefore, low opacity values indicate high transparency of the film. Measurements were taken on pTMS and TMS films (n=6).

*Fourier transform infrared (FTIR) spectroscopy*

Fourier transform infrared spectroscopy was carried out on a Bruker ALPHA II FTIR spectrometer to analyse pTMS and TMS samples. A total of 32 scans were performed in a range from 4000 to 800 cm-1 at 4 cm-1 resolution.

*Water uptake*

The hydration curve was determined by evaluating the water uptake (WU) by the film at different time points. First, dry samples of 8 mm $\varnothing$ (n= 3) were taken from the formulated film. The discs were then weighed (We) and immersed into 1 mL of distilled water at 4°C. At different study times (5', 10', 15', 30', 1 h, 1 h 30', 2 h, 2 h 30', 3 h, 3 h 30', 5 h, 5 h3 0', 6 h, 24 h, 48 h, 72 h, 96 h), samples were removed from the distilled water, the excess liquid was removed with a filter paper and the wet weight was determined. Starting from the initial weight value ($W_0$) and the weight at each study time ($W_t$). The hydration was determined for each time point by the following formula:

$$WU\ (\%) = \frac{W_t - W_0}{W_0} \times 100$$

*Water vapour transmission rate*

To analyse the permeability capacity of the material, the Water Vapour Transmission Rate (WVTR) was calculated using the Labthink PERME™ W3/0120 (Metrotec) permeability tester. For that aim, films were cut in samples (n=3) of 7.4 cm ø (test area of 33 cm$^2$) and were placed on the equipment at 38°C and 90% RH, according to ASTM E96-00. Then, WVTR was calculated with the following formula:

$$WVTR\left(\frac{g}{m^2 * day}\right) = \frac{G}{A * t}$$

where G is the weight change (g), t is time (days) and A is the test area (m2).

*Puncture test*

Puncture test was carried out to evaluate the pressure resistance of the TMS using the texture analyser TA.XTplusC equipped with a 5 kg load cell. The analysis was carried out using an aluminium cylinder of 5 mm $\varnothing$, the crosshead speed was set at 1 mm/s. Puncture tests were performed on the samples (n= 8) using a film support rig (HDP/FSR). Samples were conditioned at 100% relative humidity for 48 h prior to testing.

*Mucoadhesion test*

The mucoadhesive properties were determined using a TA.XtplusC texture analyser equipped with a 5 kg load cell, a gel mucoadhesion attachment (A/GMP) and a mucoadhesion rig (A/MUC). The maximum force required to separate the film from the membrane and the total amount of force involved in removing the film from the membrane were calculated using samples from the TMS (n= 8) soaked in 1 % porcine stomach mucin in DPBS at 37°C.

### 3. TMS in vitro characterisation

*Cell culture*

The biological evaluation of the TMS was conducted according to the recommendations of ISO 10993-5. For that, the human fibroblast cell line HS27 (ECACC) was cultured in Dulbecco's Modified Eagle's Medium (Sigma) supplemented with 10 % (v/v) inactivated foetal bovine serum (Gibco), 1 % (v/v) penicillin-streptomycin (Lonza) and 1 % (v/v) L-glutamine (Sigma) at 37°C in an incubator with an atmosphere of 5 % CO2. The culture medium was changed every 2 days and cell passages were performed weekly depending on cell confluence. At the fifth passage, the cells were collected by adding 0.25 % Trypsin-EDTA (Sigma) for 5 min and the cell suspension was centrifuged at 1500 rpm for other 5 min. The pellet obtained was resuspended in the previously described culture medium to obtain a homogeneous suspension.

*Biocompatibility*

For the biocompatibility evaluation, cells were seeded at a density of 25000 cells/cm$^2$ in 96 or 24 well plates with 100 or 500 $\mu$l of medium, respectively. After 24 h, sterile samples (5 or 8 mm $\varnothing$) of the biomaterial were placed in the wells, and some wells were leave without biomaterial to serve as controls. On the one hand, short-term biocompatibility was evaluated at 24, 48 and 72 h by the Cell-Tox Green (Promega) cytotoxicity kit and the Cell Counting Kit-8 (Sigma), following in both cases the manufacturer's protocol, and the plates were read on the GloMax Discover (Promega) and Haloled 96 (Dynamica), respectively. On the other hand, the long-term follow-up was conducted at 1, 3 and 7 days using the Live/Dead Cell Viability Assay (Thermo Fisher) kit and its guideline. Pictures were taken with the ZEISS LSM 900 confocal microscope. In addition to the study conditions, positive (live) and negative (dead) controls were included. For the former, cells seeded under the same conditions, but without biomaterial, were used. For the negative controls, in addition to not being exposed to the biomaterial, cells were treated with a lysis solution to cause cell death. For the Cell-Tox Green cytotoxicity assay, the given buffer was used, and for the CCK-8 and the Live/Dead Cell Viability Assay, DMSO (Sigma) was used as lysis agent. For short-term biocompatibility, once the results were obtained, they were related to the controls.

*Cell adhesion*

For this assay, 50000 cells/cm$^2$ were seeded on the sterilised samples (8 mm $\varnothing$) of the film, and analysed after 3, 7 and 14 days. At each time-point, samples were washed with DPBS and then fixed by Histofix (PanReac). Samples were then washed again and stored at 4°C until they were collected at all time-points. An immunofluorescence was performed by using $\alpha$-tubulin for the observation of microtubules (Abcam, 1:200) with donkey anti-mouse AF647 (Molecular probes, 1:200) as a secondary antibody, together with TRITC-labelled phalloidin for the observation of actin filaments (Sigma, 1:100). Nuclei were labelled with DAPI (Invitrogen), samples were mounted with Fluoromont (EMS) and, finally, images were taken with a ZEISS LSM 900 confocal microscope.

## 4. TMS in vivo evaluation

All the research was conducted in the Biogipuzkoa Health Research Institute (Donostia, Spain). All experiments and procedures were approved by the local ethics committee and followed the ethical principles and current legislation on protection of animals for research (Real Decreto, 2021; BOE, 2021; Directiva, 2010/63/UE). The study was performed in 10 healthy Sprague Dawley rats (Charles River Labs) of 8 weeks of age and both sexes (1:1).

*Perforation surgery and TMS placement*

First, animals were anaesthetised with 5% isoflurane (Baxter) and 1 L/min oxygen flow and the basal health status of the animals was monitored in terms of auditory response and TM appearance. Then, for the creation of the chronic perforation, mitomycin-C (0.4 mg/mL) (Sigma) was applied for 10 min using a sponge soaked in the compound, followed by the creation of a perforation of approximately 2 mm ø in the area of the pars tensa with a Wullstein scalpel. Finally, dexamethasone (4 mg/mL) (Sigma) was administered in drops for 4 consecutive doses. This procedure was performed in both ears of the 10 animals, followed by an injection of Metacam (1 mg/Kg) (Boehringer Ingelheim) as analgesia. Perforations were followed for 8 weeks by observation through the otoendoscope, which is the minimum time for considering a perforation chronic. At the eighth week, the status of the perforations was evaluated by otoendoscopic observation and otoemission test was performed to evaluate audiological function. TMs were organized into the following groups: discarded (complete or closed perforations) (n=13), control TM (no TMS placed) (n=2) or study TM (TMS placed) (n=5). In those TM chosen for film placement, the remaining epithelium was revived for cell activation and 2 mm $\varnothing$ film sample was placed, previously sterilised and hydrated with DPBS to ensure adhesion to the surrounding tissue. The TMs were followed for further 8 weeks by otoscopic evaluation when the auditory response and TM appearance were assessed before being sacrificed by inhalation of 70% CO$^2$ for TM sample extraction.

*Otoendocopic evaluation*

TM appearance and condition was monitored by otoendoscopic observation, both prior to perforation, after perforation surgery, and after TMS placement until the endpoint. For this purpose, the Olympus True view 1.9 mm 0.65 mm 30° otoendoscope (Olympus) coupled to the Karl Storz Endoskope tele pack X LED TP100 camera (Storz) was used. Specifically, images were taken after surgery on days 0, 7, 14, 21, 28, 28, 35, 35, 42, 49 and 56; and after TMS placement, on days 0, 10, 20, 30, 40, 40, 50 and 60.

From the images, the total tympanic area and the area of the perforation were measured using Imaged software. Subsequently, the closure ratio of the perforation was calculated once the TMS was placed, by relating the perforation area to the total area. In addition, the values of the different study times were normalised with respect to the value of the initial time in order to be able to compare the results between samples.

*Otoacoustic emissions test*

Distortion product otoacoustic emission (DPOAE) measurements were performed bilaterally before TM perforation, before TMS implantation, and at endpoint in order to monitor the hearing capacity of the animals throughout the study. For that, the Otodynamics Ltd. IL-O88 (Hatfield Herts) was used with a neonate probe. The measurements were performed by placing the probe in the external ear canal and maintaining the background noise lower than 50 dB. DPOAE's (2f1-f2 cubic distortion product components) were measured in the general diagnostic mode. The ratio between the f2 and f1 frequencies (f2/f1) was kept at 1.22. The amplitude of the stimulus was L1 for f1 frequency and L2 for f2 frequency. The difference between the L1-L2 levels was kept at 10 dB sound pressure level (SPL) (L1=65 dB SPL, L2=55dB SPL). The DPOAEs were measured at the 2f1-f2 frequency with the microphone in the external ear-canal and were recorded at 1001, 1501, 2002, 3003, 4004, 6006, and 7996 Hz frequencies at the geometrical means of f1 and f2. The average time needed on each test was 30 s per ear on each animal. DPOAE amplitude 3 dB higher than the noise threshold was considered meaningful. The evaluation of DPOAE results were based on signal-to-noise ratios (SNR) at 1001, 1501, 2002, 3003, 4004, 6006 and 7996 Hz frequency bands, by calculating the average and standard deviations of the DPOAE values for every single ear.

*Tissue extraction and histological evaluation*

After the sacrifice of the animals and for the extraction of the TM, first the temporal bone was resected and removed in order to expose the tympanic bulla. This was opened and the ossicular chain was removed to individualise each TM. Then, samples were cleaned of surrounding tissue, washed with PBS 1x and fixed with Histofix for 24 h. Samples were then decalcified and processed to create a paraffin block, where the TMs were placed transversely. The block was entirely cut into 5 $\mu$m slices for complete observation of the tissue. The central sample, where the handle of the malleus was observed, was taken as a reference and the pre- and post-stained samples were analysed by Haematoxylin-Eosin staining, together with the reference. Briefly, sections were dewaxed and hydrated through a series of xylene and ethanol to tap water. Sections were stained with Harris Haematoxylin (PanReac) for 8 min and then immersed into 0.1% hydrochloric alcohol in order to differentiate the Haematoxylin. After that, slides were submerged in ammoniacal water and stained with alcoholic Eosin for 1 min. Washing with running water was carried out between the different described steps. Finally, sections were dehydrated in increasing ethanol up to xylol and then mounted with DPX (Scharlab). Images were taken once the slides were completely dry with an AxioScan 7 (ZEISS). Thickness as well as cross-sectional area of the TM were measured with Imaged.

*Statistical analysis*

In order to determine significant differences between groups in the TMS characterisation, GraphPad Prism software (GraphPad 8.3.0) was used. Specifically, Kruskal-Wallis test and Dunn's multiple comparison test were conducted; $p < 0.05$ level was considered as statistical significance. Statistical differences were shown as *$p \leq 0.05$, **$p \leq 0.01$, ***$p \leq 0.001$ and ****$p \leq 0.0001$.

Results

*TMS physicochemical properties*

TMS films were easy to handle, transparent and had a brownish-orange colour, as can be seen in Figure 1A. Regarding the internal structure of the material, analysed by SEM, the construct consisted of a homogeneous and uniform architecture, where no stratifications could be distinguished within the film, as can be seen in the cross-section of the TMS in Figure 1B. Furthermore, TMS appeared to have a compact organisation in which neither clusters of material nor a highly porous configuration were evident. It is worth noting that the film thickness (Figure 1C) increased from 70 $\mu$m to 134.5 $\mu$m (p<0.001) after sterilisation.

Considering that the opacity value is calculated based on UV absorption at 600 nm, a decrease in this parameter indicates greater transparency of the sterilized film. The film showed high transparency (Figure 1D), which increased significantly (p=0.002) with the purification and sterilisation steps. Concerning the CIELAB parameters, both samples, before (L*: 78.63; a*: 9.07; b*: 62.40) and after (L*:77.23; a*:9.60; b*:51.31) sterilisation, were clear with a colour close to red and yellow, suggesting orange in a three-dimensional scale. The parameter $\Delta$E, which represents the difference in colour with respect to the white control, showed significant differences between the pre- and post-sterilised samples (p<0.001), specifically a decrease in the colour variation could be observed (Figure 1E). The roughness of the film was determined by measuring the gloss, as these parameters are inversely proportional. As can be seen in Figure 1F, the film gloss increased (p<0.0001) after sterilisation, thus decreasing the material roughness.

To study the interactions between the components that constituted the TMS, as well as the possible changes caused by the post-manufacturing processes, the FTIR spectra were evaluated for both films. As can be seen in Figure 1G, the typical protein bands could be identified around 1630, 1550 and 1240 cm$^{-1}$, corresponding to C=O stretching (amide I band), N-H bending (amide II band) and C-N stretching (amide III band), with no change after sterilisation. The main differences between non-sterilised and sterilised TMS were on the bands around 3290 cm$^{-1}$ (O-H stretching), 1012 cm-1 (C-O stretching in glycerol), and 1075 cm$^{-1}$ (C-O stretch-

ing in lactose). Specifically, a decrease of both lactose and glycerol bands and an increase in water could be observed after sterilisation. In this regard, the film proved to have great hydration capacity, especially in the first hours, as TMS increased its weight up to 96.4% after 2 h of incubation in DPBS and after that, swelling was stabilised (Figure 2A and 2B). The film also demonstrated semi-permeability properties, as observed in WVTR values (Figure 2A).

To determine the functional properties of the TMS, on the one hand, its behaviour under unilateral pressure was evaluated in an attempt to simulate the differential pressures to which the ear is exposed. On the other hand, the ability of the material to adhere to the surrounding tissue was tested to assess the possibility of avoiding suturing at the time of implantation. Figures 2C and 2D show the behaviour of a sample in both mechanical tests, and Figure 2A shows the average results for all the samples tested. In the case of the pressure resistance, the material was deformed progressively as the applied force increased until break, reaching an average force of 39.9 N at the breaking point and a deformation of 7 mm. As for mucoadhesion, 5.4 g of force were needed to detach the construct from mucin.

*TMS in vitro characterization*

The biocompatibility of the TMS was determined after exposing the human fibroblast cell line HS27 to direct contact with the film. Figure 3A shows the quantitative results of cell activity and mortality at short times. As can be seen, mortality remained at around 0% at all times of the study, while the average cell activity was above 70% in all the studied time points. Regarding the Live/Dead staining qualitative results at longer times, it could be confirmed that the cell viability was maintained throughout the week of study, with very little presence of dead cells, with a small percentage at 24 h and 7 days (Figure 3B). Moreover, cells were able to maintain the typical fibroblast phenotype while proliferating throughout the 7 days of the study without any relevant alteration. Along with biocompatibility, adhesion of the same cell type to the TMS surface was assessed up to 14 days of culture, after which immunofluorescent staining was performed for the observation of structures composing the cytoskeleton. As shown in Figure 3C, the film allowed cell adhesion and maintenance of the described cell phenotype. Together with this, cells appeared to align along the surface and develop the cytoskeletal structures according to this conformation, since it was observed that both microtubules and actin filaments correspond to the morphology of these structures in homeostasis.

*TMS in vivo characterisation*

To evaluate the integration and functionality of the designed construct, a cPTM rat model was used in which the chronic perforation was first created in the pars tensa area (Figure 4A) and then, the TMS was implanted, followed by otoscopy and evaluation of otoacoustic emissions in both phases of the study. As can be seen in Figure 4B, all perforations were created trying to generate a similar sized wound in all animals (n=20 ears). As previously mentioned, after follow-up for 8 weeks, the TMs were diagnosed and grouped. Of the total, n=8 were diagnosed as complete perforations in which no TM remained for the placement of the substitute, and n=5 of them were completely closed and were therefore excluded from the second phase of the study. Of the remaining n=7, n=2 were kept as perforation controls while n=5 of them were included in the study group for TMS placement; animals were randomised between the two groups, including perforations of different sizes in both groups. However, after epithelium revival, construct placement and cPTM follow-up, n=2 animals experienced a displacement of the TMS, exposing the wound, and were discarded from the trial. Another animal suffered from excessive cerumen accumulation and suppuration associated with cPTM maintenance and had to be excluded from the study. Regarding the animals included in the second phase of the analysis, it can be observed how TMS implantation group perforations healed after 10 days of follow-up, this being the first observation time, while the perforations in the control group remained open until the end point (Figure 4B and 4C). Those perforations treated with the construct did not seem to develop any kind of infection or inflammatory process during the follow-up, thus, at the endpoint, the treated cPTM morphologically resembled the healthy control (Figure 4A), with the three main structures, PF, PT and HM, being distinguishable. On the contrary, those animals included in the control group showed alterations on the anatomy, as the HM seemed to be lost and the PT acquired a certain opacity and whitish colour that resembled calcification areas. In addition, excessive cerumen accumulation and purulent secretion could be observed in these animals. Regarding the otoacoustic emissions test, although there were isolated significant differences, in general, no hearing alteration was observed in any animal group or study time (Figure 4D).

The histological study performed with the samples taken after 60 days of treatment confirmed the results observed in the otoendoscopic follow-up (Figure 5A), demonstrating that the TMS-treated perforations resemble the healthy control, presenting a TM of similar morphology and structure. As can be seen, the TM of the healthy control presented a keratinised thin epithelium, consisting of 1-2 keratinocyte layers. The layers of connective and mucous tissue consisted, respectively, of several layers of fibroblasts embedded in a network of collagen, and a mucous epithelium formed by voluminous cells. Concerning the TM treated with the substitute, the integrity of the tissue was verified, thereby confirming the absence of perforations. Structurally, in the first of the samples, although in the pars flaccida region it was possible to distinguish the trilaminarity of the TM, these were mainly formed by a single layer of cells. Conversely, the pars tensa region, the three main layers of the ME appeared somewhat thicker, especially the regions of epithelium and connective tissue. In the epithelium specifically, similar to the healthy control, a certain cellular stratification could be distinguished, as well as a thin layer of keratin. In this sample, it was not possible to identify the structure of the middle ear due to its loss during histological processing. In the second sample treated with the substitute, similarly; a thin central zone where the three main layers of cells are barely distinguished, and a thickened zone corresponding to the regenerative area of the TM. In this regenerative area, the connective tissue composed the main bulk of the membrane, with a high density of collagen fibers still unorganized and dispersed cells. The epithelium was only formed by a layer of cells, whereas in that regenerative region, the mucosa was formed by 2 layers of cells. Likewise, in both cases, no remnants of the implanted material were observed, and there were also no signs of giant cells, inflammation, or other indicators of a chronic response to a foreign body, such as the substitute. On the contrary, the perforation control sample showed evident hyperplasia of the TM in both the epithelium and the fibrous layer, attributed to the chronicity of the wound and attempts at regeneration. Additionally, ongoing cell infiltration and neovascularization of certain regions were observed. Moreover, several regions of calcification, as well as a scab covering the perforation, could be observed. From these histological samples, the total tympanic area was quantified, as well as the thickness of the epithelium, in order to quantitatively reflect the regeneration process. Corroborating the previous results, both the size of the area (Figure 5B) and the thickness (Figure 5C) of the TM in the TMS group were similar to that of the healthy control, while the perforation control samples showed an increase in both measures, with significant differences in the case of thickness (Ctrl. Perforation vs healthy p=0.0046; Ctrl. Perforation vs SMT p<0.0001; Ctrl. Healthy vs SMT p>0.9999).

**Claims**

1. Biomaterial film comprising:

    - A protein component, in a concentration comprised between 2 and 8 % by weight,
    - Mono/disaccharides, in a concentration comprised between 0.5 and 2 % by weight,
    - Agar, in a concentration comprised between 0.1 and 1 % by weight, and
    - Glycerol, in a concentration comprised between 0.2 and 1.5 % by weight,

    wherein the film has a thickness comprised between 70-150 $\mu$m and a rugosity lower than 70 GU.

2. The biomaterial film according to claim 1 wherein:

    - The protein component is present in a concentration comprised between 4 and 6% by weight,
    - The mono/disaccharides are present in a concentration comprised between 0.5 and 1.5 %.
    - The agar is present in a concentration comprised between 0.1-0.5 % by weight, and
    - The glycerol is present in a concentration comprised between 0.2-1% by weight.

3. The biomaterial film according to claim 1 or 2 wherein the protein component is of animal origin.

4. The biomaterial film according to claim 3 wherein the protein component is gelatin.

5. The biomaterial film according to claim 4 wherein the gelatin is selected from porcine, bovine or marine gelatin.

6. The biomaterial film according to any of claims 1 to 5 wherein the disaccharide is lactose.

7. Method to obtain the biomaterial film of claim 1 comprising the steps of:

    a) Obtaining an aqueous solution by mixing the protein component, in a concentration between 2 and 8 %, and the mono/disaccharides, in a concentration of 0.5-2%, with distilled water, Mili Q or PBS,
    b) Immersing the mixture obtained in a) into an oil bath, 50-90 °C for 15-40 min at 50-500 rpm,
    c) Obtaining an aqueous solution by mixing the agar, in a concentration between 0.1 and 1 %, with distilled water, Mili Q or PBS, and homogenizing for 5-20 min at 95-120 °C,
    d) Mixing the solutions obtained in b) and c) and adding glycerol in a concentration between 0.2 and 1.5%,
    e) Adjusting the pH of the mixture obtained in d) to a pH basic between 8-11, and continuing the homogenization for other 15-40 min at 50-90 °C,
    f) Pouring the preparation obtained in e) into moulds, and drying at room temperature for 48-96 h to obtain the films, and
    g) Incubating the films obtained in f) for 6-36 h at 50-120 °C to induce crosslinking.

8. Method according to claim 7 further comprising the steps of:

    h) Purifying the films obtained in g) by dialysis with distilled water or Mili Q for 24-72 h,
    i) sterilizing the purified films by immersion into 70 % ethanol for 5-20 min and exposing the sterilized membranes to UV radiation for 5-30 min.

9. Method according to claim 7 or 8 wherein the protein component is gelatin.

10. Method according to any of claims 7-9 wherein the disaccharide is lactose.

11. Biomaterial film according to any of claims 1-6 for use in medicine.

12. Biomaterial film according to claim 11 for use in the treatment of chronic perforations of the tympanic membrane.

13. Biomaterial film according to claim 11 for the manufacture of a tympanic membrane implant.

14. Tympanic membrane implant comprising the biomaterial film of any of claims 1-6.

15. Tympanic membrane implant according to claim 14 wherein the biomaterial film comprises:

    - 5% by weight of gelatin,
    - 1% by weight of lactose
    - 0.25% by weight of agar, and
    - 0.5% by weight of glycerol,

    wherein the film has a thickness comprised between 70-150 $\mu$m and a rugosity lower than 70 GU.

**FIG. 1A**

S4800 5.0kV 7.9mm x1.5k SE(M)      30.0um

**FIG. 1B**

**FIG. 1C**

**FIG. 1D**

**FIG. 1E**

**FIG. 1F**

**FIG. 1G**

EP 4 585 194 A1

| Physicochemical properties | | | Mechanical properties | |
| --- | --- | --- | --- | --- |
| **WU** | **WVTR** | | **PR** | **AC** |
| % | g/(m²day) | | N | g |
| 96.4 ± 5.1 | 1408.0 ± 13.2 | | 39.9 ± 2.3 | 5.4 ± 0.4 |

# FIG. 2A

FIG. 2B

FIG. 2C

FIG. 2D

EP 4 585 194 A1

FIG. 3B

FIG. 3A

FIG. 3C

Control (1)
Control (2)
TMS (1)
TMS (2)

Perforation ratio AS

2.0  1.5  1.0  0.5  0.0

Time (days)

0   10   20   30   40   50   60

**FIG. 4B**

Healthy control

PF

HM

PT

**FIG. 4A**

FIG. 4C

| Frequency (Hz) | Baseline | | Before treatment | | After treatment | |
|---|---|---|---|---|---|---|
| | Control | TMS | Control | TMS | Control | TMS |
| 1001 | $-5.55 \pm 0.20$ | $-5.36 \pm 1.27$ | $-6.67 \pm 0.04$ | $-6.33 \pm 0.76$ | $-7.11 \pm 0.14$ | $-7.23 \pm 1.10^{*}$ |
| 1501 | $-3.04 \pm 0.03$ | $-3.17 \pm 0.23$ | $-4.66 \pm 0.30^{*}$ | $-3.94 \pm 0.11$ | $-4.45 \pm 0.66$ | $-4.01 \pm 0.02$ |
| 2002 | $-3.08 \pm 0.01$ | $-3.23 \pm 0.26$ | $-3.69 \pm 0.18$ | $-3.23 \pm 0.16$ | $-3.85 \pm 0.23$ | $-3.26 \pm 0.18$ |
| 3003 | $-1.79 \pm 0.08$ | $-1.99 \pm 0.35$ | $-2.39 \pm 0.25$ | $-2.24 \pm 0.42$ | $-2.61 \pm 0.25$ | $-2.15 \pm 0.24$ |
| 4004 | $2.80 \pm 0.18$ | $2.75 \pm 0.30$ | $2.16 \pm 0.21$ | $2.06 \pm 0.37$ | $2.01 \pm 0.19$ | $2.03 \pm 0.23$ |
| 6006 | $20.12 \pm 0.47$ | $18.82 \pm 0.86$ | $19.18 \pm 0.23$ | $18.14 \pm 0.29$ | $19.72 \pm 1.46$ | $18.42 \pm 0.16$ |
| 7996 | $31.14 \pm 0.07$ | $27.33 \pm 0.45$ | $27.73 \pm 0.42^{****}$ | $27.16 \pm 1.16$ | $27.88 \pm 0.16^{****}$ | $27.75 \pm 1.30$ |

## FIG. 4D

FIG. 5A

FIG. 5B

FIG. 5C

## EUROPEAN SEARCH REPORT

**Application Number**

EP 24 38 2019

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ETXABIDE ALAITZ ET AL: "Ultra thin hydro-films based on lactose-crosslinked fish gelatin for wound healing applications", INTERNATIONAL JOURNAL OF PHARMACEUTICS, ELSEVIER, NL, vol. 530, no. 1, 5 August 2017 (2017-08-05), pages 455-467, XP085169230, ISSN: 0378-5173, DOI: 10.1016/J.IJPHARM.2017.08.001 | 1-11 | INV. A61F11/00 A61L27/20 A61L27/22 |
| A | * abstract * * page 456, paragraph 2.1 * * page 462; figure 5 * * page 461, paragraph 3.4 * * page 464, paragraph 3.10 * | 12-15 | |
| A | GARCIA-ORUE ITXASO ET AL: "Agar/gelatin hydro-film containing EGF and Aloe vera for effective wound healing", JOURNAL OF MATERIALS CHEMISTRY. B, vol. 11, no. 29, 1 January 2023 (2023-01-01), pages 6896-6910, XP093176145, GB ISSN: 2050-750X, DOI: 10.1039/D2TB02796H * abstract * * page 2, column 1, lines 19-23 * | 1-15 | TECHNICAL FIELDS SEARCHED (IPC) A61F A61L |
| A | US 11 684 695 B2 (EAR SCIENCE INST AUSTRALIA [AU]; UNIV DEAKIN [AU]) 27 June 2023 (2023-06-27) * claim 1 * * examples 2-5, 12 * | 1-15 | |

The present search report has been drawn up for all claims

| Place of search | Date of completion of the search | Examiner |
|---|---|---|
| The Hague | 19 June 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 2019

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2024

| Patent document cited in search report | | Publication date | Patent family member(s) | | Publication date |
|---|---|---|---|---|---|
| US 11684695 | B2 | 27-06-2023 | AU | 2017250021 A1 | 27-09-2018 |
| | | | CA | 3018765 A1 | 19-10-2017 |
| | | | CN | 109069699 A | 21-12-2018 |
| | | | EP | 3442610 A1 | 20-02-2019 |
| | | | JP | 7121658 B2 | 18-08-2022 |
| | | | JP | 2019511339 A | 25-04-2019 |
| | | | KR | 20190011234 A | 01-02-2019 |
| | | | US | 2020306411 A1 | 01-10-2020 |
| | | | US | 2023355839 A1 | 09-11-2023 |
| | | | WO | 2017177281 A1 | 19-10-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GROEN JJ**. Functional Anatomy of Hearing. *Acta Otolaryngol (Stockh)*, 1951, vol. 40 (1-2), 32-41
- **LIM DJ**. Structure and function of the tympanic membrane: a review. *Acta Otorhinolaryngol Belg.*, 1995, vol. 49 (2), 101-115
- **KNUTSSON J et al.** Collagen Type Distribution in the Healthy Human Tympanic Membrane. *Otol Neurotol.*, 2009, vol. 30 (8), 1225-1229
- **LIM DJ.** Human Tympanic Membrane: An Ultrastructural Observation. *Acta Otolaryngol (Stockh).*, 1970, vol. 70 (3), 176-186
- **SCHMIDT SH et al.** Tympanic-Membrane Structure - New Views. *ORL.*, 1991, vol. 53 (1), 32-36
- **STENFELDT K et al.** The Collagen Structure of the Tympanic Membrane: Collagen Types I, II, and III in the Healthy Tympanic Membrane, During Healing of a Perforation, and During Infection.. *Arch Otolaryngol Neck Surg.*, 2006, vol. 132 (3), 293
- Tympanic Membrane Perforations. **DOLHI N. et al.** StatPearls. StatPearls Publishing, 01 June 2023
- **GLADSTONE HB et al.** Tympanic membrane wound healing. An overview. *Otolaryngol Clin North Am.*, 1995, vol. 28 (5), 913-932
- **SANTA MARIA PL et al.** Histology of the healing tympanic membrane following perforation in rats: Tympanic Membrane Healing Histology. *The Laryngoscope.*, 2010, vol. 120 (10), 2061-2070
- **KIM SW et al.** Latent progenitor cells as potential regulators for tympanic membrane regeneration. *Sci Rep.*, 2015, vol. 5 (1), 11542
- **JELLINGE ME et al.** Spontaneous closure of traumatic tympanic membrane perforations: observational study.. *J Laryngol Otol.*, 2015, vol. 129 (10), 950-954
- **MASAKI M et al.** Experimental Cholesteatoma: Epidermal Ingrowth through Tympanic Membrane following Middle Ear Application of Propylene Glycol. *Acta Otolaryngol (Stockh).*, 1989, vol. 108 (1-2), 113-121
- **ORJI FT et al.** Determinants of spontaneous healing in traumatic perforations of the tympanic membrane. *Clin Otolaryngol.*, 2008, vol. 33 (5), 420-426
- **DVORAK DW et al.** Repair of chronic tympanic membrane perforations with long-term epidermal growth factor: Tympanic Membrane Repair. *The Laryngoscope.*, 1995, vol. 105 (12), 1300-1304
- **ALEEMARDANI M et al.** Can Tissue Engineering Bring H-ope to the Development of Human Tympanic Membrane?. *Tissue Eng Part 8 Rev.*, 2021, vol. 27 (6), 572-589
- **HARDMAN J et al.** Tympanoplasty for Chronic Tympanic Membrane Perforation in Children: Systematic Review and Meta-analysis. *Otol Neurotol.*, 2015, vol. 36 (5), 796-804
- **GHANAD I et al.** A Systematic Review of Nonautologous Graft Materials Used in Human Tympanoplasty. *The Laryngoscope.*, 2021, vol. 131 (2), 392-400
- **GURUSWAMY DAMODARAN R et al.** Tissue and organ decellularization in regenerative medicine. *Biotechnol Prog.*, 2018, vol. 34 (6), 1494-1505
- **SAINSBURY E et al.** Tissue engineering and regenerative medicine strategies for the repair of tympanic membrane perforations.. *Biomater Biosyst.*, 2022, vol. 6, 100046
- **HUSSAIN Z. et al.** Necessities, opportunities, and challenges for tympanic membrane perforation scaffolding-based bioengineering. *Biomed Mater.*, 2021, vol. 16 (3), 032004